(19) Europäisches Patentamt
European Patent Office
Office européen des brevets

(11) **EP 2 437 254 A1**

(12) **EUROPÄISCHE PATENTANMELDUNG**

(43) Veröffentlichungstag:
04.04.2012 Patentblatt 2012/14

(51) Int Cl.:
*G10K 11/34* (2006.01)   *A61N 7/02* (2006.01)
*A61N 7/00* (2006.01)

(21) Anmeldenummer: 11177188.7

(22) Anmeldetag: 11.08.2011

(84) Benannte Vertragsstaaten:
AL AT BE BG CH CY CZ DE DK EE ES FI FR GB GR HR HU IE IS IT LI LT LU LV MC MK MT NL NO PL PT RO RS SE SI SK SM TR
Benannte Erstreckungsstaaten:
BA ME

(30) Priorität: 29.09.2010 DE 102010041654

(71) Anmelder: Zimmer MedizinSysteme GmbH
89231 Neu-Ulm (DE)

(72) Erfinder: Bussek, Karlheinz
86459 Deubach (DE)

(74) Vertreter: Hassa, Oliver Michael
Isarpatent
Postfach 44 01 51
80750 München (DE)

(54) **Strahlungsfeldapplikator**

(57) Die Erfindung betrifft einen Strahlungsfeldapplikator zur Abgabe eines Strahlungsfeldes an einen Körper mit einer ersten Strahlung abgebenden Einrichtung, welche von einem ersten Steuersignal gespeist wird, und mindestens einer zweiten Strahlung abgebenden Einrichtung, welche von mindestens einem zweiten Steuersignal gespeist wird, wobei das erste Steuersignal gegenüber dem zweiten Steuersignal frequenzgleich und phasenverschoben ist. Die Erfindung betrifft ferner einen Strahlungsfeldapplikator.

## Fig. 2

EP 2 437 254 A1

**Beschreibung**

TECHNISCHES GEBIET

Die vorliegende Erfindung betrifft einen Strahlungsfeldapplikator für einen Körper sowie ein Verfahren zur Abgabe eines Strahlungsfeldes an einen Körper.

HINTERGRUND DER ERFINDUNG

Die Behandlung mit Strahlungsfeldern ist in der Medizin seit geraumer Zeit ein feststehender Faktor. Eingesetzt werden dabei zum Beispiel Hochfrequenz- und/oder Ultraschallsignale in verschiedenen Frequenzbereichen wie beispielsweise 27 MHz (Kurzwellentherapie), 2,4 GHz (Mikrowellentherapie) oder Ultraschall bis zu 100 kHz ("niederfrequenter Ultraschall") oder über 500 kHz ("hochfrequenter Ultraschall") mit veränderlicher Intensität von einigen Milliwatt pro Quadratzentimeter bis zu einigen Watt pro Quadratzentimeter bestrahlter oder beschallter Fläche.

Zur Zuführung des Strahlungsfeldes an einen menschlichen oder tierischen Körper werden häufig Applikatoren eingesetzt, welche das Strahlungsfeld lokal auf den zu behandelnden Körper übertragen. Beispielsweise werden für Anwendungen mit Ultraschall so genannte Ultraschallköpfe verwendet.

In diesem Zusammenhang ist es wünschenswert, bei der Behandlung mit Strahlungsfeldern die in den Körper eingebrachte Leistung möglichst genau kontrollieren zu können. Dies ist wichtig, um die zu behandelnde Körperregion nicht durch übermäßigen und unkontrollierten Wärmeeintrag zu schädigen, beispielsweise durch Verbrennungen, Zell-, Knochenhaut- oder Gewebeläsionen oder Kavitation in Körperflüssigkeiten wie beispielsweise Blut oder Lymphe.

Bei Strahlungsapplikatoren können während des Betriebes so genannte "Hotspots" auftreten, also Regionen, welche durch fehlertoleranzbedingte Ungenauigkeiten in den Applikatorsystemen entstehen und zu einer eng begrenzten starken Leistungsüberhöhung und somit lokalen Überhitzung am Applikationsort führen können. Dies gründet sich in einer vorübergehenden Inhomogenität des Strahlungsfeldes und damit einhergehenden Interferenzphänomenen. Für Ultraschallsysteme können Applikatoren anhand des so genannten Strahlinhomogenitätsverhältnis BNR (Beam Non-Uniformity Ratio) bewertet werden. Beispielsweise wird bei einem BNR von 5 und einer abgegebenen Intensität von 0,5 $W/cm^2$ ein Leistungsspitzenwert von BNR * 0,5 $W/cm^2$ = 5 * 0,5 $W/cm^2$ = 2,5 $W/cm^2$ erreicht. Für den BNR sind Werte von 5 bis 6 im Normbereich, wobei ein Wert von BNR = 1 ideal wäre. Im Bereich von Hotspots können Intensitäten von bis zu dem 30-fachen des für die Strahlungsbehandlung eingestellten Leistungseintrags erreicht werden.

Da das bestrahlte Gewebe nicht homogen ist und deshalb viele Reflexionen auftreten, treten die Hotspots nicht immer an der gleichen Stelle des Strahlenbündels auf. Ein lokale Vorhersage der Leistungsspitzen lässt sich daher nicht ohne weiteres treffen.

Eine Möglichkeit, dem Auftreten von Hotspots zu begegnen, besteht darin, den Strahlungsapplikator während der Behandlung kontinuierlich zu bewegen. Dies erfordert jedoch einen hohen Zeitaufwand durch den Therapeuten. Eine weitere Möglichkeit besteht darin, amplituden- und/oder phasenmodulierte Ultraschallsignale zu verwenden. Dies führt allerdings lediglich zu einer Amplitudenmodulation der Hotspots, nicht aber zu deren vollständiger Unterbindung. Weitere Maßnahmen betreffen die sequentielle Aktivierung mehrerer Ultraschallschwingersysteme in kurzen zeitlichen Abständen.

Auch bei der Anwendung von hochfrequenter Strahlung zur Bestrahlung von Körpern können ähnliche Phänomene auftreten. Manchmal sind diese Gegebenheiten gewünscht, zum Beispiel um einen begrenzten Zielpunkt zu bestrahlen (so genannte Fokussierung). In vielen Fällen ist es jedoch beabsichtigt, ein homogenes Strahlungsfeld zur Verfügung zu haben, um auch größere Bereiche und Flächen gleichmäßig und ohne Überhöhungen in diesem Strahlungsfeld beaufschlagen zu können.

ZUSAMMENFASSUNG DER ERFINDUNG

Es ist daher eine Aufgabe der vorliegenden Erfindung, einen Strahlungsfeldapplikator und ein Verfahren zur Applikation eines Strahlungsfeldes bereitzustellen, mit denen ein Auftreten von Hotspots effektiv unterbunden wird und/oder mit denen ein kontinuierliches Bewegen des Strahlungsfeldapplikators unnötig wird.

Erfindungsgemäß wird diese Aufgabe durch einen Strahlungsfeldapplikator mit den Merkmalen des Patentanspruchs 1 und/oder durch ein Verfahren mit den Merkmalen des Patentanspruchs 9 gelöst.

Demgemäß ist vorgesehen:

- Ein Strahlungsfeldapplikator zur Abgabe eines Strahlungsfeldes an einen Körper mit einer ersten Strahlung abgebenden Einrichtung, welche von einem ersten Steuersignal gespeist wird und mindestens einer zweiten Strahlung abgebenden Einrichtung, welche von mindestens einem zweiten Steuersignal gespeist wird, wobei das erste Steuersignal gegenüber dem zweiten Steuersignal frequenzgleich und phasenverschoben ist.

- Ein Verfahren zur Applikation eines Strahlungsfeldes an einem Körper, umfassend die Schritte des Bestrahlens des Körpers mit einem ersten Strahlungsfeld, welches durch eine erste Strahlung abgebende Einrichtung erzeugt wird und des Bestrahlens des Körpers mit einem zweiten Strahlungsfeld, welches durch eine zweite Strahlung abgebende Einrichtung erzeugt wird, wobei das zweite Strahlungsfeld gegenüber dem zweiten Strahlungsfeld frequenzgleich und phasenverschoben ist.

Ein Vorteil der vorliegenden Erfindung besteht darin, dass der Strahlungsfeldapplikator nicht mehr durch den Therapeuten oder Bediener kontinuierlich über die zu behandelnde Körperregion geführt werden muss, so dass sich eine erhebliche zeitersparnis ergibt. Ein weiterer Vorteil der Erfindung besteht darin, dass die Vermeidung von Hotspots mit dem erfindungsgemäßen Strahlungsfeldapplikator unabhängig von der Frequenz der verwendeten Strahlung ist.

Vorteilhafte Ausgestaltungen und Weiterbildungen ergeben sich aus den weiteren Unteransprüchen sowie aus der Beschreibung unter Bezugnahme auf die Figuren der Zeichnung.

Vorteilhafterweise umfasst der Strahlungsfeldapplikator ein Gehäuse, in welchem die erste Strahlung abgebende Einrichtung und die mindestens eine zweite Strahlung abgebende Einrichtung angeordnet sind. Das Gehäuse kann vorteilhafterweise ferner über eine Befestigungseinrichtung, beispielsweise eine Vakuumsaugglocke oder Ähnlichem, an dem Körper (zumindest vorübergehend) befestigt werden.

Gemäß einer bevorzugten Ausführungsform ist als Strahlungsfeld ein Ultraschallfeld vorgesehen, wobei die Strahlung abgebenden Einrichtungen Ultraschallköpfe sind.

Vorteilhafterweise umfasst der Strahlungsfeldapplikator eine Phasenschiebereinrichtung, die dazu ausgelegt ist, das mindestens eine zweite Steuersignal gegenüber dem ersten Steuersignal in der Phase einstellbar zu verschieben. Die Phase kann z. B. in einem beliebigen Winkel zwischen 0° und 180° verschoben werden.

Vorteilhafterweise umfasst der Strahlungsfeldapplikator eine Amplitudenmodulationseinrichtung, die dazu ausgelegt ist, das erste und/oder mindestens eine zweite Steuersignal in der Amplitude zu modulieren. Zusätzlich kann eine Frequenzmodulationseinrichtung vorgesehen sein, die dazu ausgelegt ist, das erste und/oder mindestens eine zweite Steuersignal in der Frequenz zu modulieren.

Weiterhin kann der Strahlungsfeldapplikator in vorteilhafter Weise eine Messeinrichtung umfassen, die dazu ausgelegt ist, die Leistung des durch die Strahlung abgebenden Einrichtungen abgegebenen Strahlungsfeldes zu messen und ein Messsignal zu erzeugen. Zusätzlich kann eine Regelungseinrichtung vorgesehen sein, die dazu ausgelegt ist, die Leistungsabgabe der Strahlung abgebenden Einrichtungen in Abhängigkeit von dem Messsignal zu regeln.

In einer bevorzugten Ausführungsform ist der Strahlungsfeldapplikator derart ausgelegt, dass die erste Strahlung abgebende Einrichtung eine erste Hauptstrahlrichtung aufweist und dass die mindestens eine zweite Strahlung abgebende Einrichtung eine zweite Hauptstrahlrichtung aufweist. Die erste Strahlung abgebende Einrichtung und die mindestens eine zweite Strahlung abgebende Einrichtung sind derart angeordnet, dass die erste Hauptstrahlrichtung und die zweite Hauptstrahlrichtung einen einstellbaren Winkel einschließen. Der einstellbare Winkel ist vorteilhafterweise zwischen 0° und etwa 10°, insbesondere etwa 1°.

Die obigen Ausgestaltungen und Weiterbildungen lassen sich, sofern sinnvoll, beliebig miteinander kombinieren. Weitere mögliche Ausgestaltungen, Weiterbildungen und Implementierungen der Erfindung umfassen auch nicht explizit genannte Kombinationen von zuvor oder im folgenden bezüglich der Ausführungsbeispiele beschriebenen Merkmale der Erfindung. Insbesondere wird der Fachmann auf Einzelaspekte als Verbesserungen oder Ergänzungen zu der jeweiligen Grundform der vorliegenden Erfindung hinzufügen.

KURZE BESCHREIBUNG DER FIGUREN

Die vorliegende Erfindung wird nachfolgend anhand der in den schematischen Figuren der Zeichnung angegebenen Ausführungsbeispiele näher erläutert. Es zeigen dabei:

Fig. 1    ein Strahlungsfeldapplikator gemäß einer Ausführungsform der Erfindung;

Fig. 2    ein Strahlungsfeldapplikator gemäß einer Ausführungsform der Erfindung in höherem Detail;

Fig. 3    ein Diagramm, welches die Phasenbeziehung zwischen Strahlungsfeldern veranschaulicht;

Fig. 4    ein Phasenschieber gemäß einer weiteren Ausführungsform der Erfindung;

Fig. 4a    ein Diagramm zur Veranschaulichung der Phasenbeziehung für den in Fig. 4 gezeigten Phasenschieber;

Fig. 5    ein Phasenschieber gemäß einer weiteren Ausführungsform der Erfindung;

Fig. 6    ein Phasenschieber gemäß einer weiteren Ausführungsform der Erfindung;

Fig. 7    ein Phasenschieber und dessen Funktionsweise gemäß einer weiteren Ausführungsform der Erfindung;

Fig. 8    die Funktionsweise eines Phasenschiebers gemäß einer weiteren Ausführungsform der Erfindung;

Fig. 9    die Funktionsweise eines Phasenschiebers gemäß einer weiteren Ausführungsform der Erfindung;

Fig. 10   ein Strahlungsfeldapplikator gemäß einer weiteren Ausführungsform der Erfindung;

Fig. 11   ein Strahlungsfeldapplikator gemäß einer weiteren Ausführungsform der Erfindung.

In den Figuren der Zeichnung sind gleiche und funktionsgleiche Elemente, Merkmale und Komponenten - sofern nichts Anderes ausgeführt ist - jeweils mit denselben Bezugszeichen versehen.

BESCHREIBUNG VON AUSFÜHRUNGSBEISPIELEN

Die in den Figuren gezeigten Beispiele und Ausführungsformen beziehen sich auf Strahlungsfeldapplikatoren, welche mit Ultraschallstrahlungsfeldern betrieben werden. Insbesondere werden hierin Strahlungsfeldapplikatoren beschrieben, welche in der Medizintechnik, insbesondere der human- oder veterinärmedizinischen therapeutischen Strahlenbehandlung eingesetzt werden. Die der Erfindung zugrunde liegenden Ideen und Konzepte können jedoch ohne Weiteres auch auf andere Gebiete, wie etwa der Materialprüfung, Qualitätsprüfung, Prüftechnik oder dergleichen, übertragen werden und beschränken sich somit nicht auf den Einsatz im Zusammenhang mit Ultraschall. Es können auch andere Felder verwendet werden, die wellenförmigen Charakter haben, wie zum Beispiel Schall, elektromagnetische Wellen, sichtbares Licht, Infrarotstrahlung, UV-Strahlung, Mikrowellen, Radiowellen oder dergleichen.

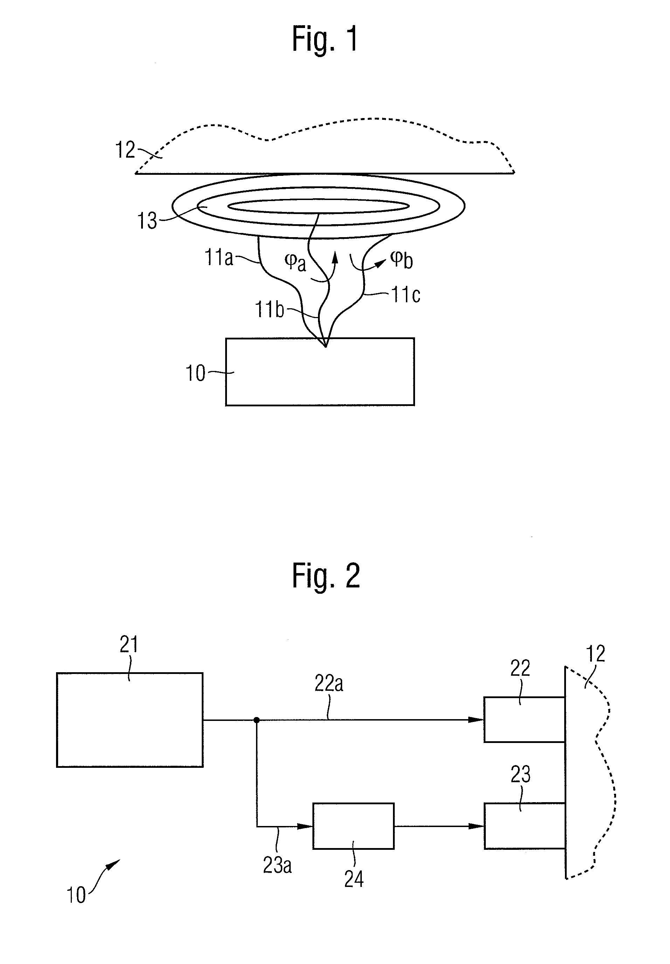

Fig. 1 zeigt einen Strahlungsfeldapplikator 10 gemäß einer Ausführungsform der Erfindung. Der Strahlungsfeldapplikator 10 gibt Strahlung 11 an einen Körper 12 ab. Die Strahlung 11 kann sich aus einzelnen Strahlungsfeldern 11a, 11b, 11c zusammensetzen, die jeweils um Phasenwinkel $\varphi_a$, $\varphi_b$ gegenüber den anderen Strahlungsfeldern verschoben sind. Das Strahlungsfeld 11a beispielsweise ist gegenüber dem Strahlungsfeld 11b um den Phasenwinkel $\varphi_a$ phasenverschoben. Das Strahlungsfeld 11b beispielsweise ist gegenüber dem Strahlungsfeld 11c um den Phasenwinkel $\varphi_b$ phasenverschoben. Die Anzahl der sich überlagernden Strahlungsfelder ist dabei beliebig groß und nicht auf die hier beispielhaft gezeigten drei Strahlungsfelder 11a, 11b, 11c begrenzt. Die Strahlungsfelder 11a, 11b, 11c überlagern sich in einem Bereich 13, welcher an oder teilweise in dem beaufschlagten Körper 12 liegt. Der Körper 12 kann beispielsweise eine Körperregion eines Menschen sein, zum Beispiel ein Oberarm, ein Fuß oder die Lendenregion. Der Körper 12 kann beispielsweise Behandlungsort für eine therapeutische Behandlung sein.

Die Strahlungsfelder 11a, 11b, 11c können von unterschiedlichen Leistungsabgabesystemen innerhalb des Strahlungsfeldapplikators 10 erzeugt werden. Dabei können über Steuersignale die Phasenwinkel $\varphi_a$, $\varphi_b$ in den Leistungsabgabesystemen variabel eingestellt werden. Durch die Interferenzen zwischen den einzelnen Strahlungsfeldern 11a, 11b, 11c in dem Bereich 13 ist somit die Entstehung von Hotspots und Inhomogenitäten unterbunden, wodurch in dem Bereich 13 ein Strahlungsfeld mit hoher Homogenität herrscht.

Fig. 2 zeigt einen Strahlungsfeldapplikator 10 gemäß einer Ausführungsform der Erfindung in höherem Detail. Der Strahlungsfeldapplikator 10 umfasst einen Generator 21, der Steuersignale für eine erste Strahlung abgebende Einrichtung 22 und eine zweite Strahlung abgebende Einrichtung 23 bereitstellt. Dazu ist der Generator 21 über Leitungen 22a und 23a mit den Strahlung abgebenden Einrichtungen 22 bzw. 23 verbunden. In der Leitung 23a ist eine Phasenschiebereinrichtung 24 angeordnet, welche das Steuersignal für die zweite Strahlung abgebende Einrichtung 23 gegenüber dem Steuersignal für die erste Strahlung abgebende Einrichtung 22 um eine Phase $\varphi$ verschiebt.

Der Generator 21 erzeugt ein Steuersignal mit einer Frequenz f, beispielsweise eine Frequenz im Ultraschallbereich von etwa 16 kHz bis etliche MHz, insbesondere zwischen 800 kHz bis 3 MHz. Das Steuersignal wird in die erste Strahlung abgebende Einrichtung 22 eingespeist. Gleichzeitig wird das Steuersignal von dem Generator 21 in eine Phasenschiebereinrichtung 24 eingespeist, in der das Steuersignal um eine Phase $\varphi$ gegenüber dem ursprünglichen Steuersignal verschoben wird. Das phasenverschobene Steuersignal wird dann in die zweite Strahlung abgebende Einrichtung 23 eingespeist. Die Strahlung abgebenden Einrichtungen 22 und 23 setzen die jeweiligen Steuersignale dann in Strahlungsfelder um, die an den Körper 12 abgegeben werden.

Der Strahlungsfeldapplikator 10 kann weiterhin eine Amplitudenmodulationseinrichtung umfassen, welche eines oder mehrere der Steuersignale für die Strahlung abgebenden Einrichtungen 22 und 23 in der Amplitude moduliert.

Der Strahlungsfeldapplikator 10 kann weiterhin eine Frequenzmodulationseinrichtung umfassen, welche eines oder mehrere der Steuersignale für die Strahlung abgebenden Einrichtungen 22 und 23 in der Frequenz moduliert.

Der Strahlungsfeldapplikator 10 kann auch ein Gehäuse umfassen, in welchem die Strahlung abgebenden Einrichtungen 22 bzw. 23 angeordnet sind.

Der Strahlungsfeldapplikator 10 kann weiterhin eine Befestigungseinrichtung umfassen, mit Hilfe derer die Strahlung abgebenden Einrichtungen 22 und 23 bzw. das Gehäuse, in dem die Einrichtungen 22 und 23 angeordnet sind, an dem Körper befestigt werden können. Die Befestigung kann vorübergehend erfolgen, beispielsweise durch eine Vakuumsaugglocke, die auf der Haut eines menschlichen Körpers angesetzt wird. Die Befestigungseinrichtung kann jede herkömmliche Befestigungseinrichtung in der Medizintechnik umfassen.

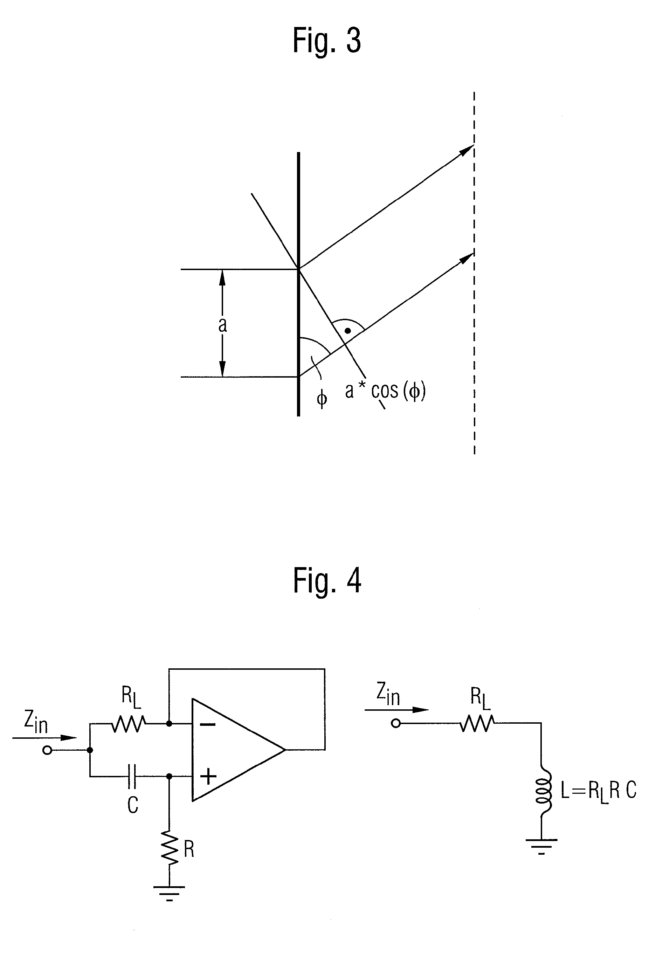

Die physikalischen Zusammenhänge zweier interferierender Strahlungsfelder sind in dem Diagramm aus Fig. 3 gezeigt. Die Überlagerung zweier Wellen gleicher Frequenz und Amplitude lässt sich anhand der trigonometrischen Additionstheoreme berechnen. Werden die beiden Wellen $f_1(t)$ und $f_2(t)$ mit der gemeinsamen Frequenz $\omega$, der Amplitude a und den Phasen $\varphi_1$ und $\varphi_2$ durch

$$f_1(t) = a * \sin(\omega t + \varphi_1)$$

und

$$f_2(t) = a * \sin(\omega t + \varphi_2)$$

beschrieben, so ergibt sich für die resultierende Überlagerung der Wellen

$$f_1(t) + f_2(t) = a * (\sin(\omega t + \varphi_1) + \sin(\omega t + \varphi_2)) =$$
$$2a * \cos(1/2(\varphi_1 - \varphi_2)) * \sin(\omega t + 1/2(\varphi_1 + \varphi_2)),$$

das heißt, es entsteht eine Welle derselben Frequenz, deren Amplitude von der Differenz der Phasen der beiden ursprünglichen Wellen abhängt und deren Phase das Mittel der Phasen der ursprünglichen Wellen ist. Für gleiche Phasen der Wellen wird der Cosinus-Term gleich 1. Es ergibt sich eine Amplitude von 2a, das heißt, die Amplitude verdoppelt sich gegenüber den Ausgangsamplituden, was konstruktiver Interferenz entspricht. Für eine Phasendifferenz von 180° wird der Cosinus-Term gleich 0, das heißt, die resultierende Welle verschwindet. Dies entspricht destruktiver Interferenz. Integriert man nun das Resultat über die Fläche, ist bei veränderlichen Phasenwinkeln das Feld ohne Hotspots und homogen.

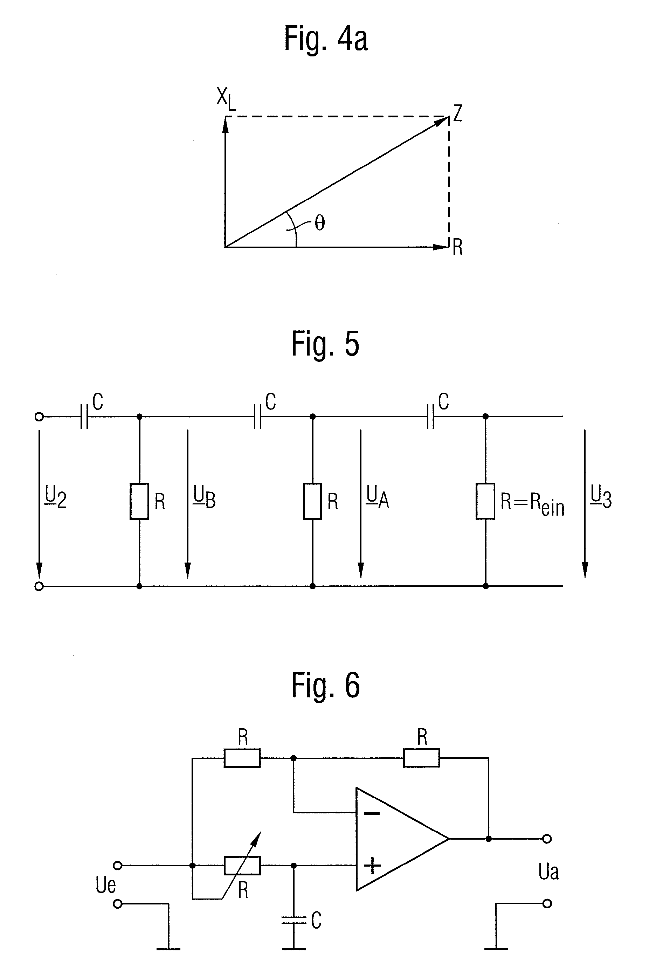

In den Figuren 4 bis 9 werden etliche von vielen Ausführungsformen für die Phasenschiebereinrichtung 24 aus Fig. 2 gezeigt. Es versteht sich, dass jede andere Phasenschiebereinrichtung ebenso eingesetzt werden kann und dass Prinzipien der Ausführungsformen der verschiedenen Figuren miteinander kombiniert werden können.

In Fig. 4 ist auf der linken Seite eine Phasenschieberschaltung mit einem Widerstand $R_L$, einer Kapazität C, einem Operationsverstärker und einem Widerstand R gezeigt. Der Widerstand $R_L$ und die Kapazität sind über den Operationsverstärker parallel geschaltet. Ein Eingang des Operationsverstärkers ist über den Widerstand R an ein Massepotential gekoppelt, der andere Eingang ist mit dem Ausgang des Operationsverstärkers rückgekoppelt.

Auf der rechten Seite in Fig. 4 ist das Ersatzschaltbild gezeigt, welches für die Phasenschieberschaltung angegeben werden kann. An einem Spannungseingang sind ein Widerstand $R_L$ und eine Induktivität L in Reihe geschaltet und mit einem Massepotential verbunden. Der komplexe Widerstand $Z_{RL}$ dieser Schaltung ergibt sich zu

$$Z_{RL} = R_L + i\omega L.$$

Die Eingangsimpedanz des Operationsverstärkers ergibt sich zu

$$Z_{in} = (R_L + i\omega R_L RC) \, || \, (R + (i\omega C)^{-1}),$$

wobei bei hinreichend großem R der erste Term dominiert. Damit entspricht der Blindwiderstand $R_L RC$ der Induktivität L. Die Induktivität L lässt sich dann durch die Größen der Elemente auf der linken Seite als $L = R_L * R * C$ ausdrücken.

Fig. 4a zeigt die Phasenbeziehung für eine RL-Reihenschaltung. Auf der x-Achse ist der reale Widerstand R, auf der y-Achse der Blindwiderstand $X_L$ aufgetragen. Je größer der Blindwiderstand $X_L$ in Relation zum realen Widerstand R ist, desto größer wird der Winkel θ (Phasenwinkel) zwischen x-Achse und komplexem Widerstand Z:

$$\theta = \tan^{-1}(X_L/R).$$

Mit variablem realen Widerstand R, zum Beispiel einem Feldeffekttransistor oder einem digitalen Potentiometer kann der Phasenwinkel θ variabel eingestellt werden.

Fig. 5 zeigt ein Schaltbild einen Phasenschieber gemäß einer weiteren Ausführungsform. Durch eine schaltbar ausgelegte Serienschaltung dreier RC-Glieder können drei Spannungen $U_B$, $U_A$ und $U_3$ abgegriffen werden, deren Phasenwinkel gegenüber der Eingangsspannung $U_2$ jeweils 60°, 120° und 180° beträgt.

Fig. 6 zeigt ein Schaltbild eines Phasenschieber gemäß einer weiteren Ausführungsform. Hier gezeigt ist ein Allpass, welcher bei tiefen Frequenzen eine geringe Phasenverschiebung zwischen Eingangsspannung $U_e$ und Ausgangsspannung $U_a$ erzeugt, bei hohen Frequenzen aufgrund der Niederohmigkeit der Kapazität C hingegen als invertierender Verstärker wirkt, also eine große Phasenverschiebung erzeugt. Der Phasenwinkel φ ist hier frequenzabhängig und berechnet sich zu

$$\varphi = -2 * \arctan(\omega R_{pot} C),$$

wobei $R_{pot}$ der variabel einstellbare Widerstand im Eingangspfad des positiven Eingangs des Operationsverstärkers ist.

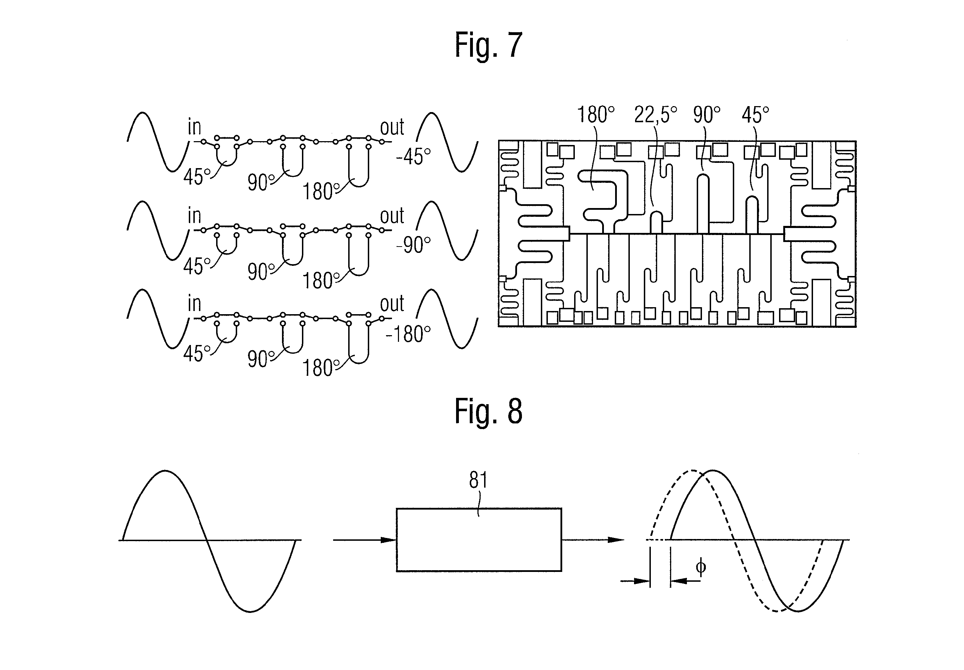

Fig. 7 zeigt einen Phasenschieber gemäß einer weiteren Ausführungsform der Erfindung. Ab einem bestimmten Frequenzbereich mit Wellenlängen im Millimeterbereich können Phasenverschiebungen auch mit Umweg- oder Laufzeitleitungen erreicht werden. Rechts im Bild ist eine analoge Leitung mit Laufzeitleitungen dargestellt, die verschiedene Phasenverschiebungen zwischen 22,5° und 180° erzeugen können, je nach Länge der Laufzeitleitung. Links im Bild sind drei verschiedene Szenarien gezeigt, die verschiedene Phasenverschiebungen erzeugen. Im ersten Beispiel wird lediglich eine 45°-Laufzeitleitung durchlaufen, so dass sich ein Phasenwinkel von -45° des Ausgangssignals gegenüber dem Eingangssignal einstellt. Im zweiten Beispiel wird lediglich eine 90°-Laufzeitleitung durchlaufen, so dass sich ein Phasenwinkel von -90° des Ausgangssignals gegenüber dem Eingangssignal einstellt. Im dritten Beispiel wird lediglich eine 180°-Laufzeitleitung durchlaufen, so dass sich ein Phasenwinkel von -180° des Ausgangssignals gegenüber dem Eingangssignal einstellt. Selbstverständlich können durch geeignete Kombinationen von Durchläufen durch Laufzeitleitungen entsprechende Phasenwinkel erzeugt werden, die als Summe der einzelnen Laufzeitleitungen gebildet werden.

In Fig. 8 ist die Funktionsweise eines Phasenschiebers gemäß einer weiteren Ausführungsform der Erfindung gezeigt. Ein Eingangssignal wird hierbei in ein analoges Laufzeitverzögerungsglied 81 eingespeist. Das analoge Laufzeitverzögerungsglied 81 kann hierbei ein sogenannter Eimerkettenspeicher oder auch ein Oberflächenwellenfilter sein. Als Resultat ergibt sich aus Ausgangssignal des analogen Laufzeitverzögerungsgliedes ein um den Phasenwinkel φ gegenüber dem Eingangssignal verschobenes Signal.

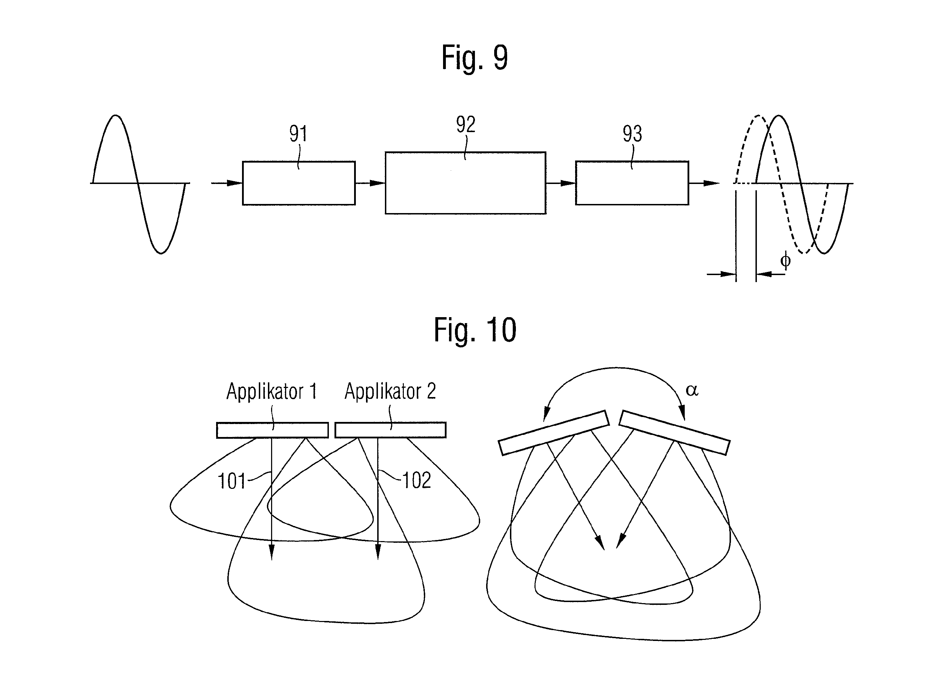

In Fig. 9 ist die Funktionsweise eines Phasenschiebers gemäß einer weiteren Ausführungsform der Erfindung gezeigt. Anstatt analoge Schaltungselemente zu verwenden, kann eine Phasenverschiebung auch durch zeitliche digitale Signalverzögerung erreicht werden. Ein Eingangssignal wird zunächst in einen Analog-Digital-Wandler 91 eingespeist, dessen digitales Ausgangssignal an ein digitales Laufzeit-Schieberegister 92 übergeben wird (First-In-First-Out-Speicher (FIFO)). Dadurch wird die Laufzeit des digitalen Signals verlängert und bei einer Rücktransformation des digitalen Ausgabesignals des digitalen Laufzeit-Schieberegisters 92 mit einem Digital-Analog-Wandler 93 wird ein analoges Ausgangssignal erzeugt, welches um den Phasenwinkel φ gegenüber dem analogen Eingangssignal verschoben ist.

Bei der Digitalisierung muss gemäß dem Nyquist-Kriterium die Abtastfrequenz mindestens doppelt so groß sein wie die Frequenz des zu digitalisierenden Signals. Als Beispiel wird zur Wahrung der Signalgüte eine Abtastfrequenz in einer Größenordnung von etwa dem 8- bis 10-fachen der Frequenz des Eingangssignals gewählt. Für Ultraschallfrequenzen von bis zu 2,4 MHz sollte die Abtastfrequenz daher etwa 24 MHz betragen. Bei einer gewünschten Phasenverschiebung von 0° bis 180° ergibt sich für eine Ultraschallfrequenz von 2,4 MHz (in Klammern die Werte für eine Ultraschallfrequenz von 800 kHz) eine Periodendauer von 1,25 μs (0,417 μs). Ein Phasenwinkel von 180° entspricht einer halben Periodendauer, also 0,625 μs (0,208 μs). Die Verzögerungszeit muss für das Laufzeitverzögerungsglied also zwischen 0 und 625 ns (0 und 208 ns) variabel einstellbar sein, um die gewünschte Phasenverschiebung zu realisieren.

Fig. 10 zeigt einen Strahlungsfeldapplikator gemäß einer weiteren Ausführungsform der Erfindung. Der Strahlungsfeldapplikator umfasst dabei zwei Strahlung abgebende Einrichtungen (Applikator 1 und Applikator 2), welche jeweils ein Hauptstrahlrichtung 101 bzw. 102 aufweisen. Entlang der Hauptstrahlrichtung wird die Hauptintensität des Strahlungsfeldes abgestrahlt (dargestellt durch die keulenförmigen Umrandungen). Werden die Einrichtungen in einer planen Eben angeordnet, so kann es besonders in den Randbereichen der Strahlungskeulen zu örtlichen Signalüberhöhungen kommen. Daher können die beiden Strahlung abgebenden Einrichtungen vorzugsweise unter einem Montagewinkel angeordnet werden, das heißt, die Strahlung abgebenden Einrichtungen können derart ausgerichtet werden, dass ihre Hauptstrahlrichtungen einen Winkel β zueinander aufweisen. Der Winkel β kann vorzugsweise einstellbar im Bereich zwischen 0° und 10° sein, insbesondere kann der Winkel β etwa 1° betragen. Ein entsprechender Komplementärwinkel α, der in Fig. 10 gezeigt ist, kann dazu 180° bis 190°, insbesondere etwa 181° betragen.

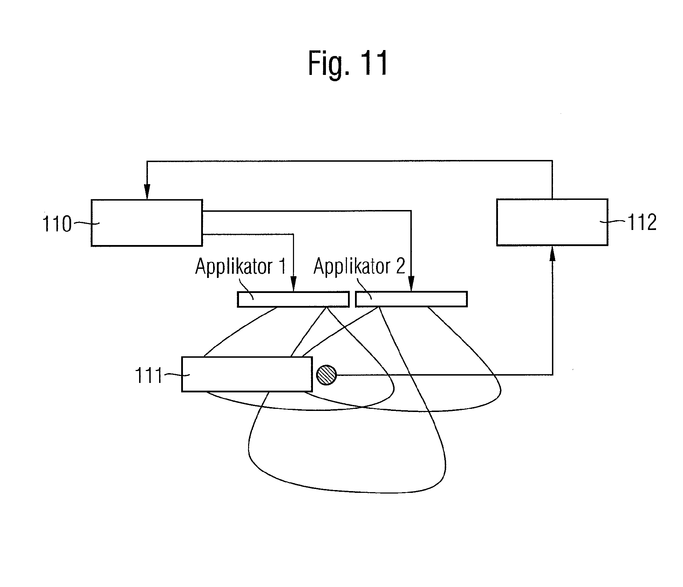

In Fig. 11 ist ein Strahlungsfeldapplikator gemäß einer weiteren Ausführungsform der Erfindung gezeigt. Der Strahlungsfeldapplikator umfasst dabei zwei Strahlung abgebende Einrichtungen (Applikator 1 und Applikator 2). Im Überlappungsbereich der beiden Strahlungsfelder der Strahlung abgebenden Einrichtungen ist eine Messeinrichtung 111, beispielsweise eine Feldsonde angeordnet, welche die Intensität und/oder die Leistung des interferierenden Strahlungsfeldes misst. Die Messeinrichtung ist dazu ausgelegt, ein Messsignal zu erzeugen, welches an eine Regelungseinrichtung 112 übergeben wird, die ein Rückkopplungssignal an eine Treibereinrichtung 110 der Strahlung abgebenden Einrichtungen einspeist. Auf diese Weise kann über eine Feedback-Schleife die Leistung im Strahlungsfeld bedarfsgerecht nachgeregelt werden.

### Patentansprüche

1.  Strahlungsfeldapplikator (10) zur Abgabe eines Strahlungsfeldes an einen Körper (12),
    mit einer ersten Strahlung abgebenden Einrichtung (22), welche von einem ersten Steuersignal gespeist wird;
    mit mindestens einer zweiten Strahlung abgebenden Einrichtung (23), welche von mindestens einem zweiten Steuersignal gespeist wird;
    wobei das erste Steuersignal gegenüber dem zweiten Steuersignal frequenzgleich und phasenverschoben ist.

2.  Applikator nach Anspruch 1, **dadurch gekennzeichnet, dass** ein Gehäuse vorgesehen ist, in welchem die erste Strahlung abgebende Einrichtung (22) und die mindestens eine zweite Strahlung abgebende Einrichtung (23) angeordnet sind.

3.  Applikator nach einem der vorherigen Ansprüche, **dadurch gekennzeichnet, dass** das Strahlungsfeld ein Ultraschallfeld ist.

4.  Applikator nach einem der vorherigen Ansprüche, **dadurch gekennzeichnet, dass** eine Phasenschiebereinrichtung (24) vorgesehen ist, die dazu ausgelegt ist, das mindestens eine zweite Steuersignal gegenüber dem ersten Steuersignal in der Phase einstellbar zu verschieben.

5.  Applikator nach einem der vorangegangenen Ansprüche, **dadurch gekennzeichnet, dass** eine Amplitudenmodulationseinrichtung, die dazu ausgelegt ist, das erste und/oder mindestens eine zweite Steuersignal in der Amplitude zu modulieren, und eine Frequenzmodulationseinrichtung, die dazu ausgelegt ist, das erste und/oder mindestens eine zweite Steuersignal in der Frequenz zu modulieren vorgesehen sind.

6.  Applikator nach einem der vorherigen Ansprüche, **dadurch gekennzeichnet, dass** eine Messeinrichtung vorgesehen ist, die dazu ausgelegt ist, die Leistung des durch die Strahlung abgebenden Einrichtungen abgegebenen Strahlungsfeldes zu messen und ein Messsignal zu erzeugen; und
dass eine Regelungseinrichtung vorgesehen ist, die dazu ausgelegt ist, die Leistungsabgabe der Strahlung abgebenden Einrichtungen in Abhängigkeit von dem Messsignal zu regeln.

7. Applikator nach einem der vorherigen Ansprüche, **dadurch gekennzeichnet, dass** die erste Strahlung abgebende Einrichtung eine erste Hauptstrahlrichtung aufweist, und die mindestens eine zweite Strahlung abgebende Einrichtung eine zweite Hauptstrahlrichtung aufweist, wobei die erste Strahlung abgebende Einrichtung und die mindestens eine zweite Strahlung abgebende Einrichtung derart angeordnet sind, dass die erste Hauptstrahlrichtung und die zweite Hauptstrahlrichtung einen einstellbaren Winkel einschließen.

8. Applikator nach einem der vorherigen Ansprüche, **dadurch gekennzeichnet, dass** eine Befestigungseinrichtung vorgesehen ist, die dazu ausgelegt ist, den Strahlungsfeldapplikator an dem Körper zu befestigen.

9. Verfahren zur Applikation eines Strahlungsfeldes an einem Körper, insbesondere mittels eines Strahlungsfeldapplikators nach einem der Ansprüche 1 bis 8, mit den Schritten:

   Bestrahlen des Körpers mit einem ersten Strahlungsfeld, welches durch eine erste Strahlung abgebende Einrichtung erzeugt wird;
   Bestrahlen des Körpers mit einem zweiten Strahlungsfeld, welches durch eine zweite Strahlung abgebende Einrichtung erzeugt wird, wobei das zweite Strahlungsfeld gegenüber dem ersten Strahlungsfeld frequenzgleich und phasenverschoben ist.

10. Verfahren nach Anspruch 9, **dadurch gekennzeichnet, dass** das erste Strahlungsfeld und das zweite Strahlungsfeld Ultraschallfelder sind.

11. Verfahren nach einem der vorherigen Verfahrensansprüche, mit den weiteren Schritten:

   Modulieren der Frequenz des ersten Strahlungsfeldes und/oder des zweiten Strahlungsfeldes; und
   Modulieren der Amplitude des ersten Strahlungsfeldes und/oder des zweiten Strahlungsfeldes.

12. Verfahren nach einem der vorherigen Verfahrensansprüche, mit den weiteren Schritten:

   Messen der Stärke des ersten Strahlungsfeldes und/oder des zweiten Strahlungsfeldes; und
   Regeln der Leistung der ersten Strahlung abgebenden Einrichtung und/oder der zweiten Strahlung abgebenden Einrichtung in Abhängigkeit von der gemessenen Stärke des ersten Strahlungsfeldes und/oder des zweiten Strahlungsfeldes.

13. Verfahren nach einem der vorherigen Verfahrensansprüchen, mit den weiteren Schritten:

   Anordnen der ersten Strahlung abgebenden Einrichtung und der zweiten Strahlung abgebenden Einrichtung in einem gemeinsamen Gehäuse; und
   Befestigen des Gehäuses an dem zu bestrahlenden Körper.

EP 2 437 254 A1

# Fig. 1

# Fig. 2

9

# Fig. 3

# Fig. 4

# Fig. 4a

# Fig. 5

# Fig. 6

Fig. 7

Fig. 8

## Fig. 9

## Fig. 10

Applikator 1   Applikator 2

EP 2 437 254 A1

# Fig. 11

Applikator 1    Applikator 2

110

112

111

Europäisches
Patentamt

European
Patent Office

Office européen
des brevets

## EUROPÄISCHER RECHERCHENBERICHT

Nummer der Anmeldung

EP 11 17 7188

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | US 2008/262350 A1 (UNGER EVAN C [US]) 23. Oktober 2008 (2008-10-23) * Absätze [0109] - [0113], [0135] - [0140]; Abbildungen 5B,11B,13 * | 1-8 | INV. G10K11/34 ADD. A61N7/02 A61N7/00 |
| X | US 2007/191906 A1 (IYER ANAND [US] ET AL) 16. August 2007 (2007-08-16) * Absätze [0025], [0029], [0033], [0039], [0043]; Ansprüche 1-4,6,7,10,34; Abbildungen 15-17 * | 1-4,6-8 | |
| X | US 2004/267118 A1 (DAWSON THOMAS PATRICK [US]) 30. Dezember 2004 (2004-12-30) * Absätze [0054], [0056], [0066]; Abbildungen 1, 4 * | 1-5,8 | |
| X | US 2002/068869 A1 (BRISKEN AXEL [US] ET AL) 6. Juni 2002 (2002-06-06) * Absätze [0088], [0089], [0095]; Ansprüche 40-41; Abbildung 15 * | 1-4,6,7 | |
| X | US 2009/112133 A1 (DEISSEROTH KARL [US] ET AL) 30. April 2009 (2009-04-30) * Absätze [0033] - [0037], [0040], [0044], [0047]; Abbildung 4B * | 1-4,6-8 | RECHERCHIERTE SACHGEBIETE (IPC) G10K A61N A61B |
| X | US 5 501 655 A (ROLT KENNETH D [US] ET AL) 26. März 1996 (1996-03-26) * Spalte 2, Zeile 13 - Spalte 5, Zeile 38; Ansprüche 23,28; Abbildungen 2,3 * | 1-5,7 | |
| X | US 2009/093724 A1 (PERNOT MATHIEU [FR] ET AL) 9. April 2009 (2009-04-09) * Absätze [0018] - [0020], [0085] - [0086], [0144] - [0145]; Abbildungen 2, 4a * | 1-4,6,7 | |

-/--

Der vorliegende Recherchenbericht wurde für alle Patentansprüche erstellt

| Recherchenort | Abschlußdatum der Recherche | Prüfer |
|---|---|---|
| München | 2. November 2011 | Link, Tatiana |

KATEGORIE DER GENANNTEN DOKUMENTE

X : von besonderer Bedeutung allein betrachtet
Y : von besonderer Bedeutung in Verbindung mit einer
anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder
nach dem Anmeldedatum veröffentlicht worden ist
D : in der Anmeldung angeführtes Dokument
L : aus anderen Gründen angeführtes Dokument

........................................................................

& : Mitglied der gleichen Patentfamilie, übereinstimmendes
Dokument

EPO FORM 1503 03.82 (P04C03)

**Europäisches Patentamt**
**European Patent Office**
**Office européen des brevets**

# EUROPÄISCHER RECHERCHENBERICHT

Nummer der Anmeldung

EP 11 17 7188

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | US 5 810 888 A (FENN ALAN J [US]) 22. September 1998 (1998-09-22) * Spalte 3, Zeile 43 - Spalte 8, Zeile 65; Abbildungen 2-3 * ----- | 1,2,4,6, 7 | |
| | | | **RECHERCHIERTE SACHGEBIETE (IPC)** |

Der vorliegende Recherchenbericht wurde für alle Patentansprüche erstellt

| Recherchenort | Abschlußdatum der Recherche | Prüfer |
|---|---|---|
| München | 2. November 2011 | Link, Tatiana |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

........................................................................................

& : Mitglied der gleichen Patentfamilie, übereinstimmendes Dokument

EPO FORM 1503 03.82 (P04C03)

**ANHANG ZUM EUROPÄISCHEN RECHERCHENBERICHT**
**ÜBER DIE EUROPÄISCHE PATENTANMELDUNG NR.**

EP 11 17 7188

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

02-11-2011

| Im Recherchenbericht angeführtes Patentdokument | Datum der Veröffentlichung | Mitglied(er) der Patentfamilie | Datum der Veröffentlichung |
|---|---|---|---|
| US 2008262350 A1 | 23-10-2008 | WO 2007058668 A1 | 24-05-2007 |
| US 2007191906 A1 | 16-08-2007 | KEINE | |
| US 2004267118 A1 | 30-12-2004 | KEINE | |
| US 2002068869 A1 | 06-06-2002 | KEINE | |
| US 2009112133 A1 | 30-04-2009 | KEINE | |
| US 5501655 A | 26-03-1996 | WO 9319705 A1 | 14-10-1993 |
| US 2009093724 A1 | 09-04-2009 | CA 2678046 A1<br>CN 101631591 A<br>EP 2121136 A1<br>FR 2912817 A1<br>WO 2008113940 A1<br>JP 2010524513 A<br>KR 20090119860 A | 25-09-2008<br>20-01-2010<br>25-11-2009<br>22-08-2008<br>25-09-2008<br>22-07-2010<br>20-11-2009 |
| US 5810888 A | 22-09-1998 | AT 285792 T<br>CA 2294196 A1<br>CN 1268059 A<br>DE 69828403 D1<br>DE 69828403 T2<br>EP 0994725 A1<br>ES 2234124 T3<br>JP 3502105 B2<br>JP 2000516841 A<br>WO 9900144 A1 | 15-01-2005<br>07-01-1999<br>27-09-2000<br>03-02-2005<br>01-12-2005<br>26-04-2000<br>16-06-2005<br>02-03-2004<br>19-12-2000<br>07-01-1999 |

EPO FORM P0461

Für nähere Einzelheiten zu diesem Anhang : siehe Amtsblatt des Europäischen Patentamts, Nr.12/82